**Europäisches Patentamt**

**European Patent Office**

**Office européen des brevets**

(19)

(11) Numéro de publication : **0 276 182 B1**

(12)

# FASCICULE DE BREVET EUROPEEN

(45) Date de publication du fascicule du brevet :
**16.10.91 Bulletin 91/42**

(51) Int. Cl.$^5$ : **C07C 309/24**

(21) Numéro de dépôt : **88400076.1**

(22) Date de dépôt : **14.01.88**

(54) **Procédé amélioré d'obtention d'alcanes sulfonamides.**

(30) Priorité : **23.01.87 FR 8700755**

(43) Date de publication de la demande :
**27.07.88 Bulletin 88/30**

(45) Mention de la délivrance du brevet :
**16.10.91 Bulletin 91/42**

(84) Etats contractants désignés :
**BE CH DE ES FR GB IT LI LU NL**

(56) Documents cités :
**DE-A- 2 821 193
US-A- 3 755 439
HOUBEN-WEYL: "Methoden der Organischen
Chemie", vol. IX, 1955, pages 398-400, Georg
Thieme Verlag, Stuttgart, DE**

(73) Titulaire : **SOCIETE NATIONALE ELF
AQUITAINE (PRODUCTION)
Tour Elf, 2, Place de la Coupole, La Défense 6
F-92400 Courbevoie (FR)**

(72) Inventeur : **Ollivier, Jean
Croix de Buzy
F-64260 Arudy (FR)**
Inventeur : **Larrouy, Michèle
1, Avenue Henri Dunant
F-64000 Pau (FR)**

(74) Mandataire : **Leboulenger, Jean et al
ATOCHEM Département Propriété Industrielle
F-92091 Paris la Défense 10 Cédex 42 (FR)**

EP 0 276 182 B1

## Description

La présente invention concerne un procédé perfectionné de préparation d'alcanes sulfonamides et, plus particulièrement, de méthane- et d'éthane-sulfonamides.

Il est connu d'obtenir du méthane sulfonamide ou ses homologues comme l'éthane- ou le butane-sulfonamide par réaction du chlorure de méthane sulfonyle ou de son homologue correspondant, avec de l'ammoniac ou une amine primaire ou secondaire. Il se forme comme sous-produit, suivant le cas, du chlorure d'ammonium ou un chlorhydrate d'amine.

Aussi a-t-on déjà préconisé d'effectuer cette réaction, en général, entre 10 et 90°C, et habituellement entre 50 et 70°C, en présence de certains diluants liquides tels que des alcools, de l'eau, des hydrocarbures aliphatiques ou aromatiques éventuellement chlorés, comme le chlorure de méthylène et le dichloréthane. Dans cet ordre d'idées, la demande publiée de brevet français n°2010654 propose, comme diluant, des nitroalcanes en $C_1$-$C_4$.

Suivant l'art antérieur, on met d'abord le chlorure d'alcane sulfonyle en solution dans le diluant, puis on introduit l'ammoniac ou l'amine dans le milieu liquide. La demande de brevet français précitée recommande de n'employer qu'un léger excès d'ammoniac ou d'amine, c'est-à-dire jusqu'à n'observer qu'une légère basicité du milieu réactionnel.

Toutefois, cette manière de procéder présente de sérieux inconvénients. Tout d'abord, elle conduit à un amide renfermant divers produits secondaires indésirés, préjudiciables à la pureté du produit utile. Dans le cas particulier de l'ammoniac comme réactif azoté, on observe la formation, entre autres, de dialkyldisulfonamine $R$-$SO_2$-$NH$-$SO_2$-$R$, ce qui implique une consommation inutile de matières premières, allant du détriment d'un bon rendement en alcanesulfonamide, sans compter les difficultés supplémentaires de séparation engendrées par la présence de ces produits secondaires.

En outre, au cours du processus de séparation et de récupération de l'alcane sulfonamide cherché, il est nécessaire de chauffer le mélange réactionnel, par exemple, entre environ 50 et 80°C, surtout lorsque la réaction proprement dite a été conduite à des températures inférieures à 50°C. Ce chauffage a pour but de solubiliser tout alcane sulfonamide resté à l'état de précipité, dans le diluant, en mélange avec le sous-produit : chlorure d'ammonium ou chlorhydrate d'amine. C'est ce qui est notamment prescrit dans la demande de brevet français n° 2010654.

De plus, les diluants proposés pour réaliser la réaction sont insatisfaisants à bien des égards. En effet, les nitroalcanes de la publication précitée ont des caractéristiques de solubilité insuffisantes à température ambiante vis-à-vis d'alcanes sulfonamides, ce qui nécessite comme on vient de l'indiquer, le maintien d'une température d'au moins 50°C afin de solubiliser le plus possible d'alcane sulfonamide. En outre, il est bien connu que les nitroalcanes sont des solvants onéreux et d'une utilisation délicate en raison de leurs limites d'explosivité assez larges. Par exemple, celles du nitrométhane sont comprises entre 7,3 et 63%.

De leur côté, les alcools ne constituent pas des milieux appropriés, n'étant pas suffisamment inertes chimiquement, puisqu'ils provoquent la formation de dialkyléthers et des esters d'acide alcanesulfonique comme produits parasites. Aussi, le sous-produit, constitué par le chlorhydrate d'amine est soluble dans les alcools, ce qui empêche sa séparation facile, par exemple par simple filtration ou centrifugation.

L'eau comme diluant donne une quantité non négligeable et indésirable d'alcane sulfonate d'ammonium.

Enfin, l'utilisation d'hydrocarbures aliphatiques ou aromatiques éventuellement chlorés, présente l'inconvénient de maintenir à l'état insoluble aussi bien les alcanes sulfonamides que les sels sous-produits, ce qui, tout comme les alcools, ne permet pas une séparation des produits de la réaction par des moyens simples de filtration ou de centrifugation.

La présente invention a pour but de remédier aux nombreux inconvénients de l'art antérieur qui viennent d'être exposés, en permettant de disposer d'un procédé fournissant des alcanes sulfonamides d'une pureté et avec un rendement élevés, tout en mettant en oeuvre des diluants chimiquement inertes facilitant la séparation et la récupération du produit utile aux alentours de la température ambiante, par des moyens simples, c'est-à-dire sans avoir, à aucun moment du procédé, à n'élever la température du milieu réactionnel au-delà de 35°-40°C.

Conformément au procédé de préparation de sulfonamides d'alcanes comportant 1 à 48 atomes de carbone, de la présente invention, on fait réagir le chlorure d'alcane sulfonyle correspondant avec de l'ammoniac ou une alkylamine primaire ou secondaire renfermant 1 à 12 atomes de carbone, en présence d'un diluant liquide sensiblement anhydre et inerte chimiquement, puis séparation et récupération du sulfonamide d'alcane désiré, procédé caractérisé en ce que l'on utilise comme diluant un ou plusieurs dialcoxyalcanes où l'alcoxy est en $C_1$-$C_3$ et l'alcane en $C_1$-$C_4$, on effectue d'abord une sursaturation du dit diluant par l'ammoniac ou l'alkylamine primaire ou secondaire avant sa mise en contact avec le chlorure d'alcane sulfonyle, et on maintient cette sursaturation tout au long de la réaction menée à une température comprise entre 10 et 40°C.

2

La demanderesse a, en effet, trouvé que lorsqu'on introduit le chlorure d'alcane sulfonyle dans un milieu liquide préalablement sursaturé en ammoniac ou en amine primaire, ou secondaire, on obtient un sulfonamide d'alcane beaucoup plus pur que celui que donne le procédé classique, ce qui se traduit par un point de fusion plus élevé du produit désiré ; cette pureté est confirmée par les analyses à l'infra-rouge, à la résonance magnétique nucléaire, la chromatographie en phase gazeuse et spectrométrie de masse.

Dans le présent exposé, on entend par le terme de "sursaturation" une présence permanente d'au moins 40% molaires et, de préférence, de 60 à 80% molaires, par rapport à la stoechiométrie de la réaction, d'ammoniac ou d'amine primaire ou secondaire, à l'état libre, dans le diluant au cours du procédé.

Selon une forme préférée de réalisation de l'invention, le diluant est constitué d'un ou de plusieurs dialcoxyalcanes où l'alcoxy est en $C_1$-$C_2$ et l'alcane en $C_1$-$C_2$.

Un groupe particulièrement satisfaisant de diluants de l'invention consiste en dialcoxyéthanes et, tout spécialement, le diméthoxyéthane, lequel est pratiquement inerte vis-à-vis des réactifs. La formation d'impuretés comme produits secondaires dans le milieu réactionnel est ainsi négligeable, du moins minime. En outre, le sel sous-produit (chlorure d'ammonium ou chlorhydrate d'amine) est essentiellement insoluble dans le diméthoxyéthane aux températures de réaction mises en oeuvre, de préférence entre 15 et 30°C. Ainsi, le diméthoxyéthane contenant l'alcane sulfonamide, en solution, peut être récupéré par évaporation ou distillation, et recyclé comme diluant frais, tandis que le sulfonamide est intégralement recueilli exempt de sous-produit.

La température de la réaction, généralement comprise entre 10° et 40°C, est plus particulièrement de 15 à 35°C, et de préférence de 20 à 30°C et encore mieux à 20°C.

Pendant toute la durée de la réaction, on introduit selon l'invention de l'ammoniac anhydre ou l'amine primaire ou secondaire, dans le diluant, avec un débit approprié. Il est avantageux d'incorporer alors le chlorure d'alcane sulfonyle de manière progressive dans le diluant préalablement sursaturé du réactif azoté.

La durée de la réaction est comprise entre 20 et 180 minutes et, de préférence, entre 60 et 80 minutes.

Il est opportun d'opérer la réaction avec un rapport molaire ammoniac ou alkylamine primaire ou secondaire/chlorure d'alcane sulfonyle d'au moins 2, et plus particulièrement, entre 3 et 5.

Bien qu'on puisse opérer la réaction sous des pressions supérieures à la pression atmosphérique, il est commode et avantageux de s'en tenir à la pression atmosphérique.

L'absence d'eau dans le milieu réactionnel est préférable, afin d'éviter des réactions secondaires indésirables. Par conséquent, le milieu réactionnel devrait être sensiblement anhydre, c'est-à-dire ne contenant pas plus de 2% en poids d'eau.

Au fur et à mesure que la réaction progresse, le sous-produit principal — chlorure d'ammonium ou chlorhydrate d'amine — apparaît sous forme d'un précipité. Le mélange réactionnel, à ce stade, se présente comme une bouillie de cristaux du sous-produit principal dans le diluant contenant en solution l'alcane sulfonamide désiré. Il est soumis à une séparation liquide-solide, selon tout moyen classique, plus particulièrement par filtration ou centrifugation.

Selon un trait particulier du procédé de l'invention, cette séparation liquide-solide est réalisée à une température comprise entre 15 et 35°C, et de préférence de 20 à 30°C.

Il est approprié de mettre en oeuvre un rapport pondéral diluant/sulfonamide d'alcane de 2/1 à 20/1 et, plus spécialement, de 3/1 à 10/1.

Le sulfonamide d'alcane est enfin séparé du diluant par tous moyens classiques tels que, par exemple, distillation ou simple évaporation sous pression atmosphérique ou sous pression réduite. On recueille l'alcane sulfonamide sous forme de poudre que l'on peut sécher. Si on le désire, on peut pousser la purification du sulfonamide par redistillation sous pression réduite.

Les exemples suivants ont pour seul but d'illustrer l'invention, et ils ne doivent donc pas être considérés comme limitant la portée de celle-ci.

## EXEMPLE 1

On fait passer de l'ammoniac anhydre dans 220 ml (191,4 g) de diméthoxyéthane (DME) comme diluant, jusqu'à sursaturation, et l'on maintient cette sursaturation pendant toute la durée de la réaction de neutralisation qui est de 60 minutes. On introduit alors 28 g de chlorure de méthane sulfonyle (CMS) en 1 heure à l'aide d'une pompe doseuse tout en maintenant un débit de 25 l/h d'ammoniac gazeux, ce qui correspond à un excès d'ammoniac de 200% molaire par rapport à l'équimolarité de la réaction de neutralisation. La température de la réaction est maintenue à environ 20°C. On produit ainsi 22,59 g de méthane sulfonamide (MSA) contenant 0,17% en poids d'ions chlore et dont le point de fusion est de 91,3°C. Le rendement est de 97,4% en ce sulfonamide, par rapport au chlorure de méthane sulfonyle engagé. Les mesures analytiques ne permettent de détecter que 500 p.p.m. en poids de méthyldisulfonamide. La pureté du produit est supérieure à 98,5%.

Les trois essais, qui suivent et qui ont été effectués d'après les méthodes de l'art antérieur, sont donnés à titre comparatif. A noter que dans les deux premiers essais, le diluant connu a été remplacé par le DME selon l'invention.

1er ESSAI

On dissout 28 g de chlorure de méthane sulfonyle (CMS) dans 220 ml de DME. On introduit alors de l'ammoniac gazeux dans ce mélange jusqu'à ce qu'il devienne légèrement basique, ce qui correspond à un excès d'NH$_3$ d'environ 10% molaire par rapport à la stoechiométrie de la réaction de formation du méthane sulfonamide. La température de réaction est d'environ 20°C. Au cours de la réaction, qui a la même durée que celle de l'exemple 1, on observe une précipitation du sous-produit chlorure d'ammonium. On filtre, et le gateau de NH$_4$Cl est lavé avec 50 ml de DME, et le liquide de lavage est mélangé au filtrat. La DME du filtrat est ensuite évaporé sous un vide partiel de 18 mm de Hg dans un évaporateur rotatif et récupéré pour être recyclé. On recueille 21,95 g de méthane sulfonamide dont le point de fusion est de 89,5°C. Le rendement est de 94,7% en ce sulfonamide par rapport au chlorure de méthane sulfonyle mis en oeuvre. L'analyse chromatographie ionique révèle la présence de 0,2% en poids d'ions chlore. En outre, l'analyse chromatographique couplée à la spectrométrie de masse met en évidence 3% en poids de méthane disulfonamide. La pureté du méthane sulfonamide n'est que de 95% contre plus de 98% pour le produit obtenu selon l'invention (exemple 1).

2ème ESSAI

On répète le 1er Essai, sauf que l'on neutralise 56 g de CMS, soit le double de la quantité du 1er Essai.

On recueille 44,5 g de méthane sulfonamide (point de fusion 89°C) d'une pureté de 94,3% avec un rendement de 95,8% en MSA. Les mesures analytiques indiquent la présence de 3,8% en poids de méthane disulfonamide (MSA).

3ème ESSAI

On procède comme dans le premier essai de l'exemple 1 ci-dessus, sauf que l'on remplace le DME par le même volume de nitroéthane.

On récupère ainsi 22,84 g d'un produit coloré en jaune et gras fondant à 87°C. Sa teneur en ions chlore est de 0,25% et le rendement en méthane sulfonamide n'est que de 90% en moles. La pureté du produit n'est que de 88%, ce qui est très insuffisant.

On remarque, de façon inattendue, que l'exemple 1, exécuté suivant le procédé de la présente invention, fournit du méthane sulfonamide d'une plus grande pureté et avec un rendement meilleur que ceux auxquels conduisent les méthodes connues.

En outre, le DME, selon l'invention, paraît supérieur en efficacité comme diluant, par rapport au nitroéthane, d'un double point de vue : on observe une meilleure solubilité du MSA notamment à basses températures (15-40°C) ; tandis qu'aux températures supérieures à 50°C, les fortes concentrations de MSA dans le nitroéthane provoquent la dissolution de NH$_4$Cl, et favorisent la formation de produits secondaires indésirés.

EXEMPLE 2

Le mode opératoire de l'exemple 1 est suivi, sauf que l'on met en oeuvre comme diluant du diéthoxyéther (DEE) sursaturé d'ammoniac, au lieu de DME. On règle le même excès d'NH$_3$ que dans l'exemple 1 tout au long de la réaction.

On produit ainsi 20,3 g de méthane sulfonamide présentant un point de fusion de 90°C. La teneur en ions chlore est de 0,111%. La pureté du produit est de 98,5%, tandis que le rendement est de 87,4%.

EXEMPLE 3

On introduit 42 g de chlorure d'éthane sulfonyle (CES) dans 330 ml de DME lequel a été préalablement sursaturé d'ammoniac anhydre avec un débit gazeux de 25 l/h de manière à établir un excès de 150% molaire d'NH$_3$ par rapport à la stoechiométrie de la réaction de neutralisation. L'introduction progressive de CES dure 1 h 30 et la température du milieu réactionnel est maintenue à 20°C. La durée de réaction est de 90 minutes.

On obtient ainsi 35 g d'éthane sulfonamide (ESA) ne contenant que 0,1% d'ions chlore. Le rendement de l'opération est de 98,2% en ESA, et la pureté du produit est de 98%.

Les deux essais suivants ont été exécutés suivant des méthodes de l'art antérieur et sont donnés à titre de comparaison.

EP 0 276 182 B1

<u>1er ESSAI</u>

On répète l'exemple 3 sauf que la température est fixée à 50°C au lieu de 20°C.
On obtient alors 36,1 g de produit brut de composition suivante :

```
Ethanè sulfonamide······  87%  (31,4g)
Ions chlore  ...........   0,2%
Ethane disulfonamide ...   9,7% en poids (3,5g)
```

Le produit est visqueux et très difficile à faire cristalliser.
Le rendement est de 88,1% en ESA (moles).

<u>2ème ESSAI</u>

On applique le mode opératoire du 1er essai à la suite de l'exemple 1, mais avec du CES et avec les mêmes proportions de réactifs que ceux indiqués dans l'exemple 3.
La composition du produit recueilli est la suivante :

```
Poids de produit brut      35,1 g
Ethane Sulfonamide         82% (28,78g)
Ions chlore                0,12%
Ethane disulfonamide       15,6% (5,47g)
```

Le rendement en ESA est de 80,75% en mole.
On constate, ici aussi, de façon surprenante, que l'ESA préparé par le procédé de l'invention présente une pureté et un rendement supérieurs à ceux que l'on obtient par les procédés utilisant des températures supérieurs à 40°C, et en n'introduisant l'$NH_3$ qu'après avoir mis le CES en contact avec le diluant.

<u>EXEMPLE 4</u>

Selon le mode opératoire de l'exemple 1, on introduit à 20°C, 28 g (0,244 M) de CMS dans 220 ml du DEE (utilisé dans l'exemple 2).
On produit ainsi 20,3 g de MSA, dont le point de fusion est de 90°C, et la pureté de 98,5%. Sa teneur en ions chlore est de 0,111%, tandis que le rendement est de 87,4%.

<u>EXEMPLE 5</u>

En suivant le mode opératoire de l'exemple 1, mais en remplaçant l'$NH_3$ par de la monoéthylamine gazeuse anhydre, on établit un excès de 60% molaire de $CH_3NH_2$ par rapport à la stoechiométrie pendant la durée de la réaction. On maintient le débit à 23 l/h de $CH_3NH_2$ dans le milieu réactionnel réglé à 20°C. On récupère ainsi 15,65 g de chlorhydrate de monométhylamine et 24,84 g de N-méthyl méthane sulfonamide, soit un rendement de 93,4% en poids. Sa teneur en ions chlore est de 1,2%. La pureté globale du produit est de 97%.

**Revendications**

1. Procédé de préparation d'alcane-sulfonamides pouvant comporter 1 à 48 atomes de carbone, consistant à faire réagir le chlorure d'alcane sulfonyle correspondant avec de l'ammoniac ou avec une alkylamine primaire ou secondaire, renfermant 1 à 12 atomes de carbone, en présence d'un diluant liquide sensiblement inerte chimiquement, puis à séparer et récupérer l'alcane-sulfonamide, caractérisé en ce que l'on utilise comme diluant un ou plusieurs dialcoxyalcanes où l'alcoxy est en $C_1$-$C_3$ et l'alcane en $C_1$-$C_4$, en ce que l'on effectue d'abord une sursaturation dudit diluant par l'ammoniac ou l'alkylamine primaire ou secondaire, avant la mise en contact avec le chlorure d'alcane-sulfonyle, et en ce que l'on maintient cette sursaturation tout au long de la réaction menée à une température comprise entre 10 et 40°C.

5

2. Procédé selon la revendication 1, caractérisé en ce que la sursaturation correspond à au moins 40% molaires et, de préférence, de 60 à 80% molaires, d'ammoniac ou d'amine primaire ou secondaire, à l'état libre, dans le diluant, par rapport à la stoechiométrie de la réaction.

3. Procédé selon la revendication 1 ou 2, caractérisé en ce que la température de réaction est plus particulièrement de 15 à 35°C, et de préférence de 20 à 30°C.

4. Procédé selon l'une des revendications 1 à 3, caractérisé en ce que la séparation et la récupération de l'alcane-sulfonamide sont effectuées à une température comprise entre 10 et 35°C et, de préférence, de 20 à 30°C.

5. Procédé selon l'une des revendications 1 à 4, caractérisé en ce que le rapport pondéral diluant/alcane-sulfonamide est de 2/1 à 20/1 et, plus spécialement, de 3/1 à 10/1.

6. Procédé selon l'une des revendications 1 à 5, caractérisé en ce que le diluant est le diméthoxyéthane.

## Patentansprüche

1. Verfahren zur Herstellung von Alkansulfonamiden, die 1 bis 48 Kohlenstoffatome enthalten können, das darin besteht, daß man das entsprechende Alkansulfonylchlorid in Gegenwart eines im wesentlichen chemisch inerten flüssigen Verdünnungsmittels mit Ammoniak oder einem 1 bis 12 Kohlenstoffatome enthaltenden primären oder sekundären Alkylamin reagieren läßt, anschließend das Alkansulfonamid abtrennt und gewinnt, dadurch gekennzeichnet, daß man als Verdünnungsmittel ein oder mehrere Dialkoxyalkane, deren Alkoxyteil 1 bis 3 und Alkanteil 1 bis 4 Kohlenstoffatome enthält, verwendet, vor dem Inberührungbringen mit dem Alkansulfonylchlorid zunächst eine Übersättigung des Verdünnungsmittels mit Ammoniak oder dem primären oder sekundären Alkylamin herbeiführt, und daß man diese Übersättigung während der gesamten Reaktion, die bei einer Temperatur zwischen 10 und 40°C durchgeführt wird, aufrechterhält.

2. Verfahren nach Anspruch 1, dadurch gekennzeichnet, daß die Übersättigung wenigstens 40 Mol% und vorzugsweise 60 bis 80 Mol% Ammoniak oder primärem oder sekundärem Amin im freien Zustand im Verdünnungsmittel, bezogen auf die Stöchiometrie der Reaktion, entspricht.

3. Verfahren nach Anspruch 1 oder 2, dadurch gekennzeichnet, daß die Reaktionstemperatur insbesondere 15 bis 35°C und vorzugsweise 20 bis 30°C beträgt.

4. Verfahren nach einem der Ansprüche 1 bis 3, dadurch gekennzeichnet, daß die Abtrennung und Gewinnung des Alkansulfonamids bei einer Temperatur zwischen 10 und 35°C und vorzugsweise von 20 bis 30°C durchgeführt werden.

5. Verfahren nach einem der Ansprüche 1 bis 4, dadurch gekennzeichnet, daß das Gewichtsverhältnis von Verdünnungsmittel/Alkansulfonamid 2/1 bis 20/1 und insbesondere 3/1 bis 10/1 beträgt.

6. Verfahren nach einem der Ansprüche 1 bis 5, dadurch gekennzeichnet, daß das Verdünnungsmittel Dimethoxyethan ist.

## Claims

1. Process for the preparation of alkanesulphonamides which may contain 1 to 48 carbon atoms, consisting in reacting the corresponding alkanesulphonyl chloride with ammonia or with a primary or secondary alkylamine containing 1 to 12 carbon atoms, in the presence of a substantially chemically inert liquid diluent, and in then separating and recovering the alkanesulphonamide, characterised in that one or more dialkoxyalkanes, where the alkoxy is $C_1$-$C_3$ and the alkane $C_1$-$C_4$, is or are employed as diluent, in that a supersaturation of the said diluent is first of all performed with ammonia or the primary or secondary alkylamine before bringing into contact with the alkanesulphonyl chloride, and in that this supersaturation is maintained throughout the reaction, which is conducted at a temperature of between 10 and 40°C.

2. Process according to Claim 1, characterised in that the supersaturation corresponds to at least 40 mol%, preferably from 60 to 80 mol%, of ammonia or of primary or secondary amine, in the free state, in the diluent, relative to the stoichiometry of the reaction.

3. Process according to Claim 1 or 2, characterised in that the reaction temperature is more particularly from 15 to 35°C and preferably from 20 to 30°C.

4. Process according to one of Claims 1 to 3, characterised in that the separation and the recovery of the alkanesulphonamide are performed at a temperature of between 10 and 35°C and preferably of 20 to 30°C.

5. Process according to one of Claims 1 to 4, characterised in that the diluent/alkanesulphonamide weight ratio is from 2/1 to 20/1 and more especially from 3/1 to 10/1.

6. Process according to one of Claims 1 to 5, characterised in that the diluent is dimethoxyethane.